# EUROPÄISCHE PATENTSCHRIFT

(11) **EP 0 959 778 B1**
(45) Veröffentlichungstag und Bekanntmachung des Hinweises auf die Patenterteilung: **07.05.2003**
(21) Anmeldenummer: 98905376.4
(22) Anmeldetag: 31.01.1998
(51) Int. Cl.: A61B 17/02

(54) **HIRNSPATEL**
BRAIN SPATULA
SPATULE POUR LE CERVEAU

(30) Priorität: 10.02.1997 DE 19704997
(43) Veröffentlichungstag der Anmeldung: 01.12.1999
(73) Patentinhaber: Aesculap AG & Co. KG, 78532 Tuttlingen (DE)
(72) Erfinder: VOM BERG, Ingo, D-78647 Trossingen (DE); HERRMANN, Gebhard, D-78597 Irndorf (DE); WEISSHAUPT, Dieter, D-78194 Immendingen (DE); WIENEKE, Paul, D-78604 Rietheim-Weilheim (DE)
(74) Vertreter: Böhme, Ulrich, Dr. Dipl.-Phys.
(86) Internationale Anmeldenummer: EP9800514
(87) Internationale Veröffentlichungsnummer: WO98034544

(56) Entgegenhaltungen:
- EP-A- 0 143 124
- DD-A- 215 468
- US-A- 3 882 855
- US-A- 4 263 900
- US-A- 4 945 896

## Beschreibung

Die Erfindung betrifft einen Hirnspatel mit einem bandförmigen Streifen aus biegbarem und nach einer Verbiegung seine Form haltendem Material, der von einem weichen und elastisch verformbaren, eine gleichmäßige Druckverteilung der Retraktionskräfte ermöglichenden sowie eine druckspitzenfreie Anpassung der Oberfläche an die Gehirnsubstanz gewährleistenden Material umhüllt ist, das an den Seitenkanten des Streifens seitlich über den Streifen vorsteht und dessen Materialstärke von den Seitenkanten des Streifens zum Rand hin abnimmt, so daß dessen Verformbarkeit von den Seitenkanten des Streifens zum Rand des weichen Materials hin zunimmt.

Hirnspatel sind in der Neurochirurgie schon lange in Gebrauch, um das Hirnparenchym zu retrahieren und so einen Kanal in die Tiefe des Operationsfeldes freizuhalten. Die häufigste Bauform ist ein schlichter Blechstreifen aus einem per Hand biegsamen Metallstreifen, dessen Oberfläche sich jedoch auf dem Gehirn bereits nach wenigen Minuten deutlich abzeichnet. Besonders auffallend sind die Abdruckmarken der seitlichen Spatelkanten, die auf eine hohe lokale Druckbelastung schließen lassen. Diese Druckbelastung kann zu entsprechenden Verletzungen führen.

Es ist bekannt, Hirnspatel im Bereich der Kanten abzurunden, um dadurch die Druckbelastung zu reduzieren. Es gelingt jedoch durch diese Maßnahme nicht, die Verletzungen in dem gewünschten Maße zu vermeiden.

Auch Versuche, das Hirnspateltrauma durch Kunststoffoberflächenbeschichtung zu entschärfen, verliefen ergebnislos.

Aus der EP-A-143124 ist ein elastisches Paddel zum Aufschieben auf das steife Blatt eines Retraktors bekannt. Der Streifen besteht aus einem nicht biegbaren Material, so daß dieses Paddel nicht ohne weiteres an die Form des zurückzuhaltenden Gewebes angepaßt werden kann.

Weiterhin ist aus der DD-A-215468 ein Wundoffenhalter in Form eines allgemein bekannten Langenbeckschen Wundhakens bekannt, jedoch mit Kunststoffüberzug und gekrümmtem Querschnitt. Auch dieser Wundoffenhalter ist für die Anlage am weichen Hirngewebe nicht geeignet.

Der nächste Stand der Technik nach US-A-3 882 855 ist im Oberbegriff von Patentanspruch 1 gewürdigt.

Es ist Aufgabe der Erfindung, einen gattungsgemäßen Hirnspatel so auszubilden, daß hohe lokale Druckbelastungen vermieden werden können.

Diese Aufgabe wird bei einem Hirnspatel der eingangs beschriebenen Art erfindungsgemäß dadurch gelöst, daß der Querschnitt des weichen Materials im Bereich des Streifens durch nach außen gewölbte bogenförmige Begrenzungslinien begrenzt wird, daß die bogenförmige Begrenzungslinie sich auf einer Seite des weichen Materials über dessen gesamte Breite erstreckt und daß sich die bogenförmige Begrenzungslinie auf der gegenüberliegenden Seite nur über einen zentralen Bereich erstreckt und beidseitig in geradlinige, im wesentlichen parallel zum Streifen verlaufende Begrenzungslinien übergeht.

Eine solche Ausgestaltung ermöglicht eine gleichmäßige Druckverteilung der Retraktionskräfte und gewährleistet damit eine Anpassung der Spateloberfläche an die Gehirnsubstanz ohne Druckspitzen. Trotzdem kann die Formstabilität des Spatels insgesamt durch den in das weiche Material eingebetteten Streifen erreicht werden. Dabei ergibt sich durch die bogenförmige Begrenzung des weichen Materials eine abgerundete, nach außen gewölbte Kontur, die zwischen sich den formstabilen Streifen einbettet. Insgesamt ergibt sich also ein stetiger, kanten- und vorsprungsfreier Verlauf, wobei sich die Materialstärke in dem seitlich neben dem formstabilen Streifen liegenden Randstreifen reduzieren läßt, so daß zum Rand hin eine erhöhte Verformbarkeit erreicht werden kann.

Es ist vorteilhaft, wenn die Gesamtbreite des weichen Materials mindestens doppelt so groß ist wie die Breite des Streifens, so daß neben dem formstabilen Streifen ein genügend breiter Bereich bleibt, in dem nur weiches Material vorgesehen ist und in dem die Verformbarkeit allmählich zunimmt.

Insbesondere kann vorgesehen sein, daß die Gesamtbreite des weichen Materials bis zu fünfmal so groß ist wie die Breite des Streifens.

Insbesondere können die Begrenzungslinien des Querschnittes des weichen Materials an den Rändern spitzwinklig zusammenlaufen, beispielsweise unter einem Winkel zwischen 10° und 30°.

Die seitlichen Randstreifen wirken daher wie seitliche Flügel oder Dichtlippen, die vom Streifen nach außen hin immer geringer werdende Retraktionkräfte übertragen, wobei diese Kräfte zum Rand hin durch den spitzwinkligen Verlauf kontinuierlich auf Null absinken.

Das weiche Material kann im Bereich des Streifens eine Schichtdicke aufweisen, die mindestens so groß ist wie die Dicke des Streifens. Dadurch wird auch in diesem Bereich durch ein elastisches Zusammendrücken des weichen Materials eine Anpassung an die Kontur des Gehirns ermöglicht und damit ein optimaler Druckausgleich.

Alternativ oder zusätzlich kann vorgesehen sein, daß die Shore-Härte des weichen Materials von den Seitenkanten des Streifens zum Rand hin abnimmt. Auch diese Maßnahme dient dazu, die Verformbarkeit des weichen Materials vom Streifen zum Rand hin zu erhöhen.

Beispielsweise kann die Shore-Härte im Bereich der Seitenkanten zwischen 50 und 70 liegen, im Bereich des Randes zwischen 20 und 40.

Insbesondere kann das weiche Material aus Silikon bestehen.

Bei einer bevorzugten Ausführungsform ist vorgesehen, daß der Streifen an einem Ende mit einem Griffteil verbunden ist.

Dieses Griffteil kann bandförmig ausgebildet sein und eine Breite aufweisen, die größer ist als die Breite des Streifens. Insbesondere entspricht die Breite des Griffteils im wesentlichen der Breite des weichen Materials.

Die nachfolgende Beschreibung bevorzugter Ausführungsformen der Erfindung dient im Zusammenhang mit der Zeichnung der näheren Erläuterung. Es zeigen:

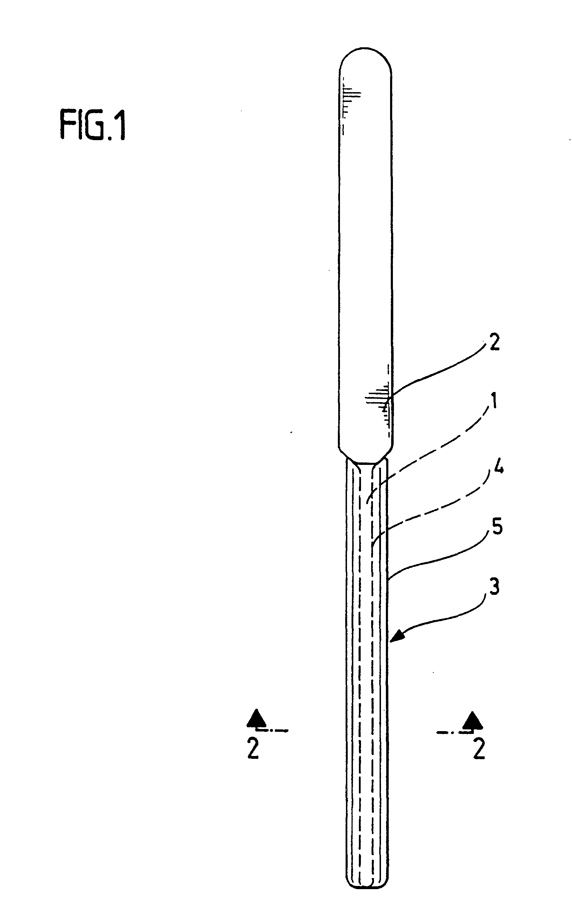

- Figur 1 :: eine Draufsicht auf ein erstes bevorzugtes Ausführungsbeispiel eines Hirnspatels;
- Figur 2 :: eine Schnittansicht längs Linie 2-2 in Figur 1;
- Figur 3 :: eine Ansicht ähnlich Figur 2 bei einem Hirnspatel geringerer Breite;
- Figur 4 :: eine Ansicht ähnlich Figur 2 bei einem Hirnspatel mit schmalerem Streifen;
- Figur 5 :: eine Ansicht ähnlich Figur 2 bei einem Hirnspatel mit einer größeren Breite des weichen Materials und
- Figur 6 :: eine Ansicht ähnlich Figur 2 mit einer noch größeren Breite des weichen Materials.

Der in der Zeichnung dargestellte Hirnspatel umfaßt einen schmalen, länglichen Streifen 1 aus einem biegsamen und formstabilen Material, beispielsweise aus Edelstahl oder aus Titan. Formstabil bedeutet dabei lediglich, daß dieser Streifen nach einer Verbiegung seine Form hält, es versteht sich also, daß dieses Material von Hand biegbar ist.

Der Streifen hat vorzugsweise einen rechteckigen Querschnitt, grundsätzlich könnte aber auch eine andere Querschnittsform Anwendung finden.

An einem Ende geht der Streifen 1 in ein breiteres, bandförmiges Griffteil 2 über, das einstückig mit dem Streifen 1 ausgebildet sein kann, das aber auch ein selbständiges Bauteil sein kann, das mit dem Streifen 1 verbunden wird.

Der Streifen 1 ist eingebettet in einen Mantel 3 aus weichem Material, beispielsweise aus Silikon. Dieser Mantel 3 umgibt den Streifen 1 auf allen Seiten, im Bereich der Oberseite und der Unterseite des Streifens 1 ist der Mantel 3 mit einer Schichtdicke ausgeführt, die etwa der Dicke des Streifens 1 entspricht. An den Seitenkanten 4 des Streifens 1 beginnend setzt sich der Mantel 3 seitlich bis zu seinem Rand 5 über eine Breite fort, die mindestens der halben Breite des Streifens 1 entspricht, die aber wesentlich größer sein kann, beispielsweise kann der Mantel 3 insgesamt fünfmal so breit sein wie der Streifen 1.

Der Mantel 3 wird an einer Seite durch eine bogenförmige, nach außen gewölbte Begrenzungslinie 6 begrenzt, die sich über die gesamte Breite des Mantels 3 erstreckt, auf der gegenüberliegenden Seite durch eine in entgegengesetzte Richtung gewölbte bogenförmige Begrenzungslinie 7, die jedoch nur im Bereich des Streifens 1 und in den beidseitig daran anschließenden Grenzbereichen nach außen gewölbt ist, während sie in den weiter außen liegenden Randbereichen in geradlinige Abschnitte 8 übergeht, die sich parallel zum Streifen 1 bis zum Rand 5 erstrecken. Der Übergang zwischen der gebogenen Begrenzungslinie 7 und den geradlinigen Abschnitten 8 erfolgt dabei nicht stufenförmig, sondern sanft.

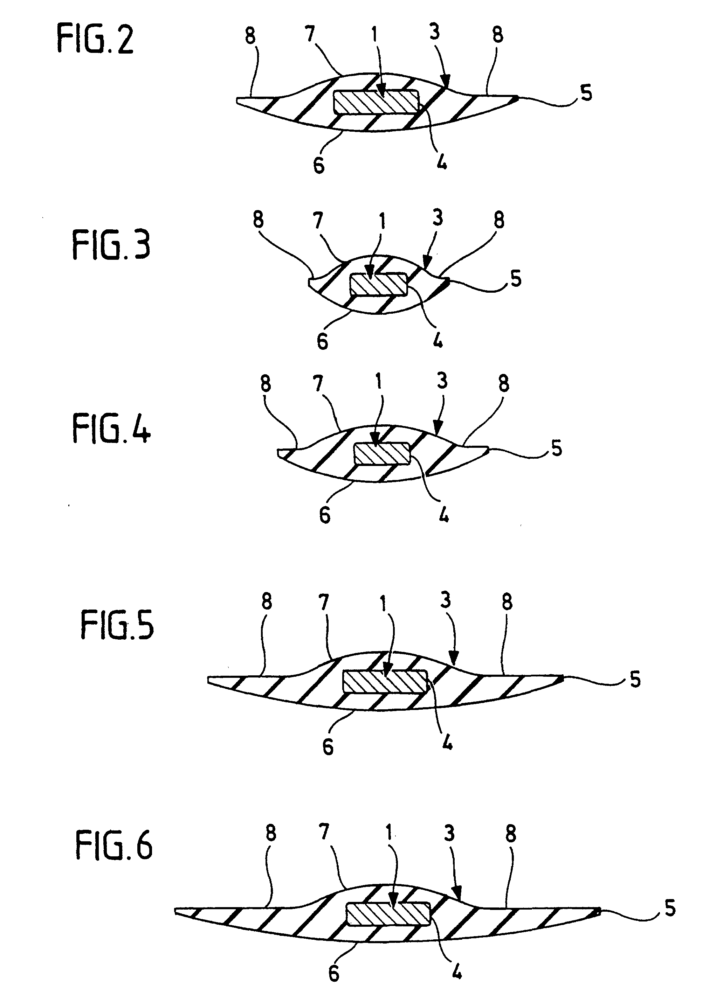

Im Bereich des Randes 5 laufen die Begrenzungslinie 7 und die Abschnitte 8 spitzwinklig zusammen, der Winkel kann zwischen 10° und 30° liegen. Die Breite des Streifens 1 und die Breite des Mantels 3 können unterschiedlich gewählt werden, in den Ausführungsbeispielen der Figuren 2 bis 6 sind unterschiedliche Dimensionierungsmöglichkeiten dargestellt.

Gemeinsam ist allen Ausführungsbeispielen, daß die Materialstärke des Mantels 3 von den Seitenkanten 4 zum Rand 5 hin abnimmt, so daß dadurch eine vergrößerte Verformbarkeit in Richtung auf den Rand 5 erreicht wird. Diese vergrößerte Verformbarkeit bedeutet auch, daß die übertragbaren Retraktionkräfte vom Streifen 1 zum Rand 5 hin abnehmen, so daß insgesamt eine vergleichmäßigte Druckeinleitung in das anliegende Gehirn erreicht wird.

Die erhöhte Verformbarkeit wird bei den bisher beschriebenen Ausführungsbeispielen ausschließlich dadurch erreicht, daß die Materialstärke des Mantels 3 zum Rand 5 hin abnimmt.

Alternativ dazu könnte auch vorgesehen sein, daß die Shore-Härte des Materials des Mantels 3 von der Mitte zum Rand 5 hin abnimmt. Dies könnte bei gleichbleibender Dicke des Mantels 3 erfolgen, besonders vorteilhaft wäre es aber, wenn diese Maßnahme kombiniert wird mit einer Abnahme der Materialdicke zum Rand 5 hin. Es könnte beispielsweise ein Material verwendet werden, das im Bereich der Seitenkante Shore-Härten zwischen 50 und 70 aufweist, im Bereich des Randes 5 dagegen Shore-Härten zwischen 20 und 40.

## Patentansprüche

1. Hirnspatel mit einem bandförmigen Streifen (1) aus biegbarem und nach einer Verbiegung seine Form haltendem Material, der von einem weichen und elastisch verformbaren, eine gleichmäßige Druckverteilung der Retraktionskräfte ermöglichenden sowie eine druckspitzenfreie Anpassung der Oberfläche an die Gehirnsubstanz gewährleistenden Material (3) umhüllt ist, das an den Seitenkanten (4) des Streifens (1) seitlich über den Streifen (1) vorsteht und dessen Materialstärke von den Seitenkanten (4) des Streifens (1) zum Rand (5) hin abnimmt, so daß dessen Verformbarkeit von den Seitenkanten (4) des Streifens (1) zum Rand (5) des weichen Materials (3) hin zunimmt, **dadurch gekennzeichnet, daß** der Querschnitt des weichen Materials (3) im Bereich des Streifens (1) durch nach außen gewölbte bogenförmige Begrenzungslinien (6, 7) begrenzt wird, daß die bogenförmige Begrenzungslinie (6) sich auf einer Seite des weichen Materials (3) über dessen gesamte Breite erstreckt und daß sich die bogenförmige Begrenzungslinie (7) auf der gegenüberliegenden Seite nur über einen zentralen Bereich erstreckt und beidseitig in geradlinige, im wesentlichen parallel zum Streifen (1) verlaufende Begrenzungslinien (8) übergeht.

2. Hirnspatel nach Anspruch 1, **dadurch gekennzeichnet, daß** die Gesamtbreite des weichen Materials (3) mindestens doppelt so groß ist wie die Breite des Streifens (1).

3. Hirnspatel nach einem der Ansprüche 1 oder 2, **dadurch gekennzeichnet, daß** die Gesamtbreite des weichen Materials (3) bis zu fünfmal so groß ist wie die Breite des Streifens (1).

4. Hirnspatel nach einem der voranstehenden Ansprüche, **dadurch gekennzeichnet, daß** die Begrenzungslinien (6, 8) des Querschnittes des weichen Materials (3) an den Rändern (5) spitzwinklig zusammenlaufen.

5. Hirnspatel nach Anspruch 4, **dadurch gekennzeichnet, daß** der Winkel zwischen den Begrenzungslinien (6, 8) zwischen 10° und 30° liegt.

6. Hirnspatel nach einem der voranstehenden Ansprüche, **dadurch gekennzeichnet, daß** das weiche Material (3) im Bereich des Streifens (1) eine Schichtdicke aufweist, die mindestens so groß ist wie die Dicke des Streifens (1).

7. Hirnspatel nach einem der voranstehenden Ansprüche, **dadurch gekennzeichnet, daß** die Shore-Härte des weichen Materials (3) von den Seitenkanten (4) des Streifens (1) zum Rand (5) hin abnimmt.

8. Hirnspatel nach Anspruch 7, **dadurch gekennzeichnet, daß** die Shore-Härte im Bereich der Seitenkanten (4) zwischen 50 und 70 liegt.

9. Hirnspatel nach Anspruch 7 oder 8, **dadurch gekennzeichnet, daß** die Shore-Härte im Bereich des Randes (5) zwischen 20 und 40 liegt.

10. Hirnspatel nach einem der voranstehenden Ansprüche, **dadurch gekennzeichnet, daß** das weiche Material (3) aus Silikon besteht.

11. Hirnspatel nach einem der voranstehenden Ansprüche, **dadurch gekennzeichnet, daß** der Streifen (1) an einem Ende mit einem Griffteil (2) verbunden ist.

12. Hirnspatel nach Anspruch 11, **dadurch gekennzeichnet, daß** der Griffteil (2) bandförmig ausgebildet ist und eine Breite aufweist, die größer ist als die Breite des Streifens (1).

13. Hirnspatel nach Anspruch 12, **dadurch gekennzeichnet, daß** die Breite des Griffteils (2) im wesentlichen der Breite des weichen Materials (3) entspricht.

14. Hirnspatel nach einem der voranstehenden Ansprüche, **dadurch gekennzeichnet, daß** der Streifen (1) aus Titan besteht.

## Claims

1. A brain spatula with a band-like strip (1) made of a material which is flexible and retains its shape following deformation, the strip being covered by a soft and elastically deformable material (3) which makes an even pressure distribution of the retraction forces possible and ensures a pressure-peak-free adaptation of the surface to the brain substance, the material (3) projecting laterally beyond the strip (1) at the lateral edges thereof and having a material strength which decreases from the lateral edges (4) of the strip (1) towards the margin (5) with the result that its deformability increases from the lateral edge (4) of the strip (1) towards the margin (5) of the soft material (3), **characterised in that** the cross section of the soft material (3) is delimited in the region of the strip (1) by outwardly arching arcuate delimiting lines (6, 7), that the arcuate delimiting line (6) extends over the full width of the soft material (3) on one side thereof, and that the arcuate delimiting line (7) on the opposite side extends over only a central region and changes bilaterally into straight delimiting lines (8) following a course substantially parallel to the strip (1).

2. A brain spatula according to Claim 1, **characterised in that** the full width of the soft material (3) is at least twice as great as the width of the strip (1).

3. A brain spatula according to one of Claims 1 or 2, **characterised in that** the full width of the soft material (3) is up to five times as great as the width of the strip (1).

4. A brain spatula according to one of the preceding claims, **characterised in that** the delimiting lines (6, 8) of the cross-section of the soft material (3) come together to form a point at the margins (5).

5. A brain spatula according to Claim 4, **characterised in that** the angle between the delimiting lines (6, 8) is between 10° and 30°.

6. A brain spatula according to one of the preceding claims, **characterised in that** the soft material (3) in the region of the strip (1) has a layer thickness at least as great as the thickness of the strip (1).

7. A brain spatula according to one of the preceding claims, **characterised in that** the Shore hardness of the soft material (3) decreases from the lateral edges (4) of the strip (1) towards the margin (5).

8. A brain spatula according to Claim 7, **characterised in that** the Shore hardness in the region of the lateral edges (4) is between 50 and 70.

9. A brain spatula according to Claim 7 or 8, **characterised in that** the Shore hardness in the region of the margin (5) is between 20 and 40.

10. A brain spatula according to one of the preceding claims, **characterised in that** the soft material (3) consists of silicone.

11. A brain spatula according to one of the preceding claims, **characterised in that** the strip (1) is connected to a handle part (2) at one end.

12. A brain spatula according to Claim 11, **characterised in that** the handle part (2) is in the form of a band and has a width greater than the width of the strip (1).

13. A brain spatula according to Claim 12, **characterised in that** the width of the handle part (2) substantially corresponds to the width of the soft material (3).

14. A brain spatula according to one of the preceding claims, **characterised in that** the strip (1) consist of titanium.

## Revendications

1. Spatule pour le cerveau, comportant une bande (1), en forme de ruban en un matériau qui est flexible et qui conserve sa forme après avoir été recourbé, qui est entourée par un matériau (3) mou et élastiquement déformable, assurant une répartition régulière de la pression des forces de rétraction ainsi qu'une adaptation sans pointe de pression de la surface à la substance cérébrale, matériau qui fait saillie sur les arêtes latérales (4) de la bande (1) latéralement au-delà de la bande (1) et dont l'épaisseur diminue depuis les arêtes latérales (4) de la bande (1) vers le bord (5) de sorte que sa déformabilité augmente depuis les arêtes latérales (4) de la bande (1) vers le bord (5) du matériau mou, **caractérisée en ce que** la section transversale du matériau (3) mou est limitée dans la région de la bande (1) par des lignes de limitation (6, 7) de forme arquée, cambrées vers l'extérieur, **en ce que** la ligne de limitation (6) de forme arquée s'étend sur un côté du matériau (3) mou sur toute sa largeur, et **en ce que** la ligne de limitation (7) de forme arquée ne s'étend sur le côté opposé que sur une région centrale et se transforme des deux côtés en des lignes de limitation (8) rectilignes s'étendant sensiblement parallèlement à la bande (1).

2. Spatule pour le cerveau selon la revendication 1, **caractérisée en ce que** la largeur totale du matériau (3) mou est au moins deux fois plus grande que la largeur de la bande (1).

3. Spatule pour le cerveau selon l'une ou l'autre des revendications 1 et 2, **caractérisée en ce que** la largeur totale du matériau (3) mou est jusqu'à cinq fois plus grande que la largeur de la bande (1).

4. Spatule pour le cerveau selon l'une des revendications précédentes, **caractérisée en ce que** les lignes de limitation (6, 8) de la section transversale du matériau (3) mou convergent en angle aigu sur les bords (5).

5. Spatule pour le cerveau selon la revendication 4, **caractérisée en ce que** l'angle entre les lignes de limitation (6, 8) est entre 10° et 30°.

6. Spatule pour le cerveau selon l'une des revendications précédentes, **caractérisée en ce que** le matériau (3) mou présente dans la région de la bande (1) une épaisseur de couche qui est au moins aussi grande que l'épaisseur de la bande (1).

7. Spatule pour le cerveau selon l'une des revendications précédentes, **caractérisée en ce que** la dureté Shore du matériau (3) mou diminue depuis les arêtes latérales (4) de la bande (1) vers le bord (5).

8. Spatule pour le cerveau selon la revendication 7, **caractérisée en ce que** la dureté Shore du matériau (3) mou dans la région des arêtes latérales (4) est entre 50 et 70.

9. Spatule pour le cerveau selon l'une ou l'autre des revendications 7 et 8, **caractérisée en ce que** la dureté Shore dans la région du bord (5) est entre 20 et 40.

10. Spatule pour le cerveau selon l'une des revendications précédentes, **caractérisée en ce que** le matériau (3) mou est en silicone.

11. Spatule pour le cerveau selon l'une des revendications précédentes, **caractérisée en ce que** la bande (1) est reliée à une extrémité avec une partie de préhension (2).

12. Spatule pour le cerveau selon la revendication 11, **caractérisée en ce que** la partie de préhension (2) est réalisée en forme de ruban et présente une largeur qui est plus grande que la largeur de la bande (1).

13. Spatule pour le cerveau selon la revendication 12, **caractérisée en ce que** la largeur de la partie de préhension (2) correspond sensiblement à la largeur du matériau (3) mou.

14. Spatule pour le cerveau selon l'une des revendications précédentes, **caractérisée en ce que** la bande (1) est en titane.
